# EUROPÄISCHE PATENTSCHRIFT

(11) **EP 2 994 680 B1**
(45) Veröffentlichungstag und Bekanntmachung des Hinweises auf die Patenterteilung: **10.07.2019**
(21) Anmeldenummer: 14725656.4
(22) Anmeldetag: 07.05.2014
(51) Int. Cl.: F16L 13/10, F16L 21/08, F16L 37/098

(54) **VERBINDUNGSSYSTEM UND VERFAHREN ZUM VERBINDEN VON FLUIDFÜHRENDEN BAUTEILEN**
CONNECTING SYSTEM AND METHOD FOR CONNECTING FLUID-CONDUCTING COMPONENTS
SYSTÈME DE LIAISON ET PROCÉDÉ DE LIAISON D'ÉLÉMENTS DE GUIDAGE DE FLUIDES

(30) Priorität: 08.05.2013 DE 102013208548
(43) Veröffentlichungstag der Anmeldung: 16.03.2016
(73) Patentinhaber: LABOMATIC INSTRUMENTS AG, CH-4123 ALLSCHWIL (CH)
(72) Erfinder: MARCHAND, Claude, CH-4434 Hölstein (CH)
(74) Vertreter: Manitz Finsterwald Patent- und Rechtsanwaltspartnerschaft mbB
(86) Internationale Anmeldenummer: PCT/EP2014/059345
(87) Internationale Veröffentlichungsnummer: WO 2014/180909

(56) Entgegenhaltungen:
- EP-A1- 0 499 819
- EP-A1- 2 048 425
- WO-A1-2005/022022
- DE-A1- 4 331 848
- DE-A1-102008 024 360
- DE-U1- 20 320 665
- FR-A1- 2 829 219
- GB-A- 2 217 419
- US-A- 4 035 005
- US-A- 5 447 341

## Beschreibung

Die vorliegende Erfindung betrifft ein Verbindungssystem zum Verbinden von zwei fluidführenden Bauteilen, wie z.B. einem ersten und einem zweiten Schlauch oder einem Schlauch und einem Anschlussteil oder einem ersten und einem zweiten Rohr, wobei das Verbindungssystem ein erstes Bauteil mit einem ersten Verbindungsbereich und ein zweites Bauteil mit einem zweiten Verbindungsbereich, der mit dem ersten Verbindungsbereich zur Ausbildung einer Verbindung kooperiert, umfasst, wobei im ersten Verbindungsbereich ein erster Strömungskanal und im zweiten Verbindungsbereich ein zweiter Strömungskanal ausgebildet ist. Ferner betrifft die Erfindung ein Verbindungssystem zum Verbinden von zwei fluidführenden Bauteilen, wobei ein freier Endbereich des einen Bauteils in einen freien Endbereich des zweiten Bauteils einsteckbar ist, sowie ein Verfahren zum Verbinden von zwei fluidtransportierenden Bauteilen.

Zur Analyse von Flüssigkeiten, z.B. mittels Chromatographie, werden fluidführende Bauteile mittels einer Steckverbindung miteinander verbunden. Solch eine Steckverbindung besteht z.B. aus einem Schlauch und einer Hohlnadel, wie beispielsweise in der EP 0 978 292 beschrieben. Beim Verbinden der Bauteile wird ein meist konisch geformtes Nadelende in den Schlauch eingeschoben, um diesen unter einer Aufweitung seines Durchmessers auf die Nadel zu schieben und durch Reibung dort zu fixieren. Beim Aufschieben des Schlauches wird zum einen das Material des Schlauches gedehnt, was zu einer Schwächung des Schlauches im Bereich der Verbindung führt, und zum anderen kann auch etwas von dem Schlauchmaterial durch die Nadel abgekratzt werden, was zu einer Kontamination bzw. Verstopfung der Nadel und/oder gar zu einer Leckage im Bereich der Verbindung führen kann.

Des Weiteren wird beim Formen der Steckverbindung ein Verschleppungsbereich (auch Totwasserbereich genannt) zwischen Nadelende und Schlauch geformt. Solche Bereiche sind Bereiche, in denen eine Flüssigkeitsströmung verschleppt wird, das heißt, dass z.B. eine Flüssigkeit oder Kontamination aus einer vorhergehenden Analyse in diesem Bereich abgelagert werden kann und nicht oder nur schlecht bei einem Spülvorgang herausgespült wird, aber sich gegebenenfalls mit einer Flüssigkeit einer späteren Analyse mischt, was zu einer Verfälschung der Analyse führen kann.

Ein weiterer Ansatz, solche Verschleppungsbereiche zu umgehen, besteht darin, verschraubbare Fittings einzusetzen. Hierbei wird ein Innengewinde an der Nadel in einem Spritzgussvorgang ausgebildet, und ein Fitting mit einem entsprechenden Außengewinde umfasst lose den Schlauch. Damit der Schlauch nicht aus dem Fitting gezogen werden kann und der Strömungskanal zwischen Nadel und Schlauch dicht ist, wird das Schlauchende im rechten Winkel zur Längsachse des Schlauchs umgeformt und eine Dichtung zwischen dem umgeformten Schlauchende und dem Fitting vorgesehen. Beim Umformen des Endes des Schlauchs entsteht ein kleiner Radius am Schlauchende, der zu einem Verschleppungsbereich zwischen dem Ende der Nadel und dem umgeformten Schlauchende führt.

Des Weiteren können die Strömungskanäle des Schlauches und der Nadel oft nicht exakt koaxial angeordnet werden, was zu weiteren Verschleppungsbereichen und zu einer Querschnittverengung des Strömungskanals in diesem Bereich beim Übergang vom Nadelende zum Schlauchende führen kann.

Stand der Technik gemäß dem Oberbegriff des Anspruchs 1 wird in der GB 2 217 419 A gelehrt. Weiterer Stand der Technik ist aus den folgenden Druckschriften bekannt:
US 5,447,341 A, DE 203 20 665 U1, WO 2005/022022 A1, DE 10 2008 024360 A1,
FR 2 829 219 A1, EP 2 048 425 A1, DE 43 31 848 A1, EP 0 499 819 A1, und US 4 035 005.

Es ist daher die Aufgabe der vorliegenden Erfindung, eine Verbindung vorzuschlagen, die zu keiner oder wenigstens keiner nennenswerten Querschnittverengung an dem Übergang zwischen den Strömungskanälen der beiden Bereiche führt und die Größe der Verschleppungsbereiche vollständig oder zumindest weitestgehend minimiert. Des Weiteren soll die Verbindung zu keiner nennenswerten Schwächung des Materials der Bauteile führen und technisch hochwertig und kostengünstig zu realisieren sein.

Zur Lösung dieser Aufgabe wird ein Verbindungssystem nach Anspruch 1 und ein Verfahren nach Anspruch 14 vorgeschlagen.

Solch ein Verbindungssystem weist ein erstes Bauteil mit einem ersten Verbindungsbereich und ein zweites Bauteil mit einem zweiten Verbindungsbereich, der mit dem ersten Verbindungsbereich zur Ausbildung einer Verbindung kooperiert, auf, wobei im ersten Verbindungsbereich ein erster Strömungskanal und im zweiten Verbindungsbereich ein zweiter Strömungskanal ausgebildet ist. Im verbundenen Zustand des Verbindungssystems sind die zwei Strömungskanäle miteinander ausgerichtet und gehen mit einem konstantem Querschnitt oder einem sich kontinuierlich verjüngenden Querschnitt mindestens des einen Verbindungsbereiches ineinander über, d.h. sie gehen zumindest im Wesentlichen ohne eine Diskontinuität ineinander über. Im Bereich der Verbindung ist zumindest ein Teil des ersten und zumindest ein Teil des zweiten Verbindungsbereichs durch eine Ummantelung vorzugsweise fixierend und gegebenenfalls auch dichtend verbunden.

Dadurch dass die zwei Strömungskanäle im verbundenen Zustand des Verbindungssystems miteinander ausgerichtet sind und mit einem konstanten Querschnitt oder einem sich kontinuierlich verjüngenden Querschnitt mindestens des einen Verbindungsbereiches ineinander übergehen, wird eine Verbindung bereit gestellt, die keine Querschnittverengung an dem Übergang zwischen den Strömungskanälen der beiden Verbindungsbereiche herbeiführt.

Die Verschleppungsbereiche werden durch einen konstanten Querschnitt oder einen sich kontinuierlich verjüngenden Querschnitt auch vollständig vermieden oder zumindest weitestgehend minimiert, so dass keine Totwasserbereiche entstehen, die im Betrieb der Verbindung zu ungewollten Störungen führen.

Ferner wird nun eine Verbindung zwischen zwei Bauteilen ausgebildet, die von außen ummantelt wird, um die Verbindung der Bauteile so zu fixieren, dass sie ausgerichtet gehalten und nicht oder wenigstens nicht ohne weiteres gelöst werden kann. Vorzugsweise verhindert die Fixierung auch, dass eine etwaige Abdichtung zwischen den zwei Bauteilen verloren geht, darüber hinaus kann auch die Ummantelung für eine Abdichtung bzw. eine zusätzliche Abdichtung der Verbindung sorgen. Die Verbindungsbereiche der jeweiligen Bauteile sind derart gefertigt, dass diese aufeinander derart abgestimmt sind, dass Verschleppungsbereiche nicht vorkommen oder weitestgehend minimiert werden und ein im Wesentlichen durchgehender innerer Querschnitt der Bauteile im Bereich der Verbindung sichergestellt wird.

Vorzugsweise bildet die Ummantelung eine nicht lösbare Verbindung der ersten und zweiten Flansche. Dies bedeutet, dass die Verbindung dauerhaft ist und insbesondere, dass durch die Ummantelung eine verbesserte Dichtigkeit der Verbindung erzeugt wird, da keine Flüssigkeit durch die Ummantelung hindurch diffundieren kann. Wenn die Ummantelung in einem Spritzgussverfahren hergestellt wird, wird ein Werkstoff, z.B. ein flüssiger Werkstoff, typischerweise in eine Spritzgussform unter Druck und/oder erhöhter Temperatur eingeführt und der Werkstoff verfestigt sich in der Spritzgussform, um die Verbindung zwischen den zwei Bauteilen herum, sodass die Ummantelung quasi ein einteiliges Verbindungssystem mit den zwei Bauteilen bildet.

Die kooperierende Verbindung zwischen dem ersten Verbindungsbereich und dem zweiten Verbindungsbereich ist vorzugsweise so ausgestaltet, dass die Verbindung dicht ist, d.h. keine Flüssigkeit bzw. Gas im Bereich der verbundenen Verbindungsbereiche austreten kann.

In einer bevorzugten Ausführungsform ist der erste Verbindungsbereich in den zweiten Verbindungsbereich einsteckbar. Dadurch, dass die Bauteile zusammensteckbar sind, wird gewährleistet, dass kein Spiel zwischen den Bauteilen vorhanden ist, was zu dem Entstehen von Verschleppungsbereichen bzw. einer Querschnittsveränderung führt. Ferner wird hierdurch eine zusätzliche Abdichtung im Verbindungsbereich durch die ineinander gefügten beispielsweise konusförmigen Teile geschaffen.

Nach einer weiteren bevorzugten Ausführungsform umfasst der erste Verbindungsbereich einen von einem freien Endbereich des ersten Bauteils zurückgesetzten Flansch und der zweite Verbindungsbereich einen am oder hinter einem freien Endbereich des zweiten Bauteils ausgebildeten Flansch.

Die Verwendung von Flanschen als Verbindungsbereiche der Bauteile ermöglicht ein noch genaueres Verbinden der zwei Bauteile, was die Wahrscheinlichkeit eines Entstehens von Verschleppungsbereichen bzw. Querschnittsveränderungen noch besser verhindert. Solche Flansche können integral mit dem jeweiligen Bauteil ausgebildet werden oder nachträglich auf das jeweilige Bauteil aufgebracht werden. Typische Beispiele solcher Flansche sind vorgefertigte Schlauchaufweitungen an Schläuchen oder Rohren und Halteflansche an Nadeln oder Armaturen. Beispielsweise könnten eine Nadel oder ein starres Bauteil wie ein Rohr mit einem externen Gewinde versehen werden, auf das der Flansch aufgeschraubt wird. Bei einem Schlauch kann der Flansch durch axiales Quetschen des Schlauches gegebenenfalls unter Erwärmung des Schlauches im Quetschbereich ausgebildet werden. Alternativ kann der Endbereich des Schlauches beispielsweise während der Ausbildung der internen konusförmigen Form gebördelt werden, um einen im Durchmesser vergrößerten Kragen zu erzeugen, der selbst als Flansch oder als Anschluss bzw. Anschlag für einen aufschiebbaren Flanschteil dient.

Gemäß der Erfindung ist eine Festhalteeinrichtung vorgesehen, die an oder hinter dem ersten Flansch und/oder an oder hinter dem zweiten Flansch angreift, um das erste und zweite Bauteil axial aneinander zu halten.

Erfindungsgemäß ist der erste Verbindungsbereich ein von einem freien Endbereich des ersten Bauteils zurückgesetzter Flansch und der zweite Verbindungsbereich ein am oder hinter einem freien Endbereich des zweiten Bauteils ausgebildeter Flansch. Durch die Verwendung von Flanschen als Verbindungsbereiche kann eine verbesserte Ausrichtung der zwei Strömungskanäle im Bereich des Übergangs geschaffen werden, wobei die Wahrscheinlichkeit von Querschnittsveränderungen im Bereich des Übergangs noch weiter minimiert wird.

Mittels einer Festhalteeinrichtung können die zwei Verbindungsbereiche bzw. die zwei Flansche der Bauteile aneinander fixiert werden. Ein Fixieren der Bauteile garantiert eine noch bessere Abdichtung zwischen den Bauteilen. Ferner gewährleistet eine solche Festhalteeinrichtung auch nach der Ummantelung, dass die zwei Bauteile nicht relativ zueinander verrutschen können, was zu einer unerwünschten Entstehung von Verschleppungsbereichen bzw. Querschnittsveränderungen führen würde.

In einem weiteren Aspekt weist die Erfindung ein Verbindungssystem zum Verbinden von zwei fluidführenden Bauteilen gemäß Anspruch 5 auf. Solch ein Verbindungssystem ermöglicht ein Verbinden von zwei fluidführenden Bauteilen, wie einem ersten und einem zweiten Schlauch oder einem Schlauch und einem Anschlussteil oder einem ersten und einem zweiten Rohr, wobei ein freier Endbereich des einen Bauteils in einen freien Endbereich des zweiten Bauteils ein-steckbar ist, so dass ein erster Strömungskanal im ersten Verbindungsbereich und ein zweiter Strömungskanal im zweiten Verbindungsbereich miteinander ausgerichtet sind. Hierbei weist das Verbindungssystem zumindest die folgenden Merkmale auf:
- einen Flansch am ersten Bauteil, der vom freien Endbereich des ersten Bauteils zurückgesetzt ist,
- einen Flansch am zweiten Bauteil, der am oder hinter dem freien Endbereich des zweiten Bauteils ausgebildet ist, sowie
- eine Festhalteeinrichtung, die an oder hinter dem ersten Flansch und/oder an oder hinter dem zweiten Flansch angreift, um das erste und zweite Bauteil axial aneinander zu halten.

Besonders bevorzugte Ausführungsformen der Erfindung sind in den Unteransprüchen und den Zeichnungen beschrieben.

Nach einer bevorzugten Ausführungsform ist die Festhalteeinrichtung durch zumindest eine Klammer ausgebildet, mittels derer die Komponenten des Verbindungssystems aneinander geklemmt werden. Vorzugsweise ist die zumindest eine Klammer an einem Ringteil ausgebildet, das hinter dem ersten Flansch oder dem zweiten Flansch, vorzugsweise axial verschiebbar hinter dem ersten Flansch oder dem zweiten Flansch, angeordnet ist. Die zumindest eine Klammer könnte aber auch an einem der Bauteile ausgebildet sein und so konzipiert sein, dass sie das andere Bauteil entweder direkt oder mittels eines weiteren Bauteils fixiert. Die Klammer könnte auch die Form eines im Querschnitt C-förmigen Rohrteils aufweisen mit nach innen ragenden Ringabschnitten an den entgegengesetzten Enden, die hinter den Flanschen greifen. Hierdurch kann die Klammer durch Spreizung des C-förmigen Rohrteils, ähnlich einem Federring, über die Verbindung geschoben werden.

Die Klammer kann aus einem Kunststoff oder aus Metall bestehen, wobei eine Klammer aus Metall durch einen Stanzvorgang aus einem Blechteil hergestellt werden kann. Hierdurch kann ein Blechteil mit Federeigenschaften verwendet werden, und die Dicke der Klammer kann auf ein Minimum reduziert werden.

Eine Klammer, die gegebenenfalls vorgespannt ist, kann so einfach nach dem Zusammenfügen der Bauteile an mindestens einem Bauteil angreifen und dieses an dem Angriffspunkt fixieren, so dass die Bauteile nicht gegeneinander verrutschen können. Vorzugsweise besteht die Festhalteeinrichtung aus mehreren Klammern, so dass die Bauteile nicht nur an einem Punkt, sondern an mehreren Punkten gehalten bzw. fixiert werden können. Beispielsweise können zwischen 1 bis 20 Klammern in Abhängigkeit der Größe der Bauteile eingesetzt werden, z.B. für Teflonschläuche mit einem Außendurchmesser im Bereich von 2,5 bis 5 mm werden vorzugsweise zwischen drei und sieben solcher Klammern eingesetzt, um den Schlauch am Flansch des ersten Bauteils zu fixieren.

Gemäß einer bevorzugten Ausführungsform ist eine Hülse am zweiten Bauteil hinter bzw. über dem zweiten Flansch angeordnet, und die Festhalteeinrichtung bzw. die zumindest eine Klammer greift an der Hülse an.

Mittels der Hülse kann ein zweites Bauteil noch besser an dem ersten Bauteil gehalten werden, insbesondere wenn zumindest die Innenform der Hülse an die Außenform des zweiten Flansches angepasst ist, da somit die Hülse Druck auf den zweiten Flansch ausüben kann und diesen an den Flansch des ersten Bauteils drücken kann, um ein gegebenenfalls vorher vorhandenes Spiel zwischen den Bauteilen zu unterdrücken und eine unerwünschte Entstehung von Verschleppungsbereichen bzw. Querschnittsveränderungen zu verhindern.

Vorzugsweise ist eine Ummantelung vorgesehen, die das erste Bauteil, das zweite Bauteil sowie die Festhalteeinrichtung bzw. die zumindest eine Klammer zumindest bereichsweise ummantelt. Hierbei werden die Bauteile von außen zusätzlich mittels der Ummantelung gestützt und abgedichtet. Die Ummantelung kann auf verschiedene Arten realisiert werden. Beispielsweise kann die Ummantelung aus einem Kunststoff oder einem Klebstoff, z.B. einem Zweikomponenten-Klebstoff bestehen oder als Schrumpfschlauch realisiert werden. Die Ummantelung kann unabhängig von ihrer Ausbildung über die gesamte Verbindung einschließlich der freiliegenden Bereiche der Bauteile oder nur über einen ausgewählten Bereich der Verbindung angebracht werden. Sollten weitere Komponenten, wie z.B. eine Hülse, zusätzlich vorgesehen sein, um die Verbindung zu verbessern, würde die Ummantelung auch die Hülse zumindest bereichsweise abdecken.

Nach einer besonders bevorzugten Ausführungsform greift die Ummantelung dichtend am ersten und zweiten Bauteil an und/oder hält die Festhalteeinrichtung bzw. die zumindest eine Klammer fest.

Die Ummantelung sorgt also für eine zusätzliche Abdichtung der Bauteile von außen. Ferner bewirkt die Ummantelung eine Stärkung der Verbindung des Verbindungssystems, da die Verbindung nun mit zusätzlichem Material von außen versehen ist, das dichtend und auch haftend die Verbindung umgibt und schützt. Vorzugsweise werden die freien Endbereiche der Ummantelung verjüngt ausgebildet, so dass ein geschmeidiger, ein Abknicken der Bauteile bzw. des Schlauches verhindernder Übergang zwischen Ummantelung und den Bauteilen entsteht.

In einer bevorzugten Ausführungsform ist die Ummantelung in einem Spritzgussverfahren ausgebildet. Ummantelungen, die im Spritzgussverfahren hergestellt werden, sind kostengünstig, relativ schnell und einfach herzustellen. Des Weiteren kann die Größe der Verbindung flexibel an die Größe der verwendeten Komponenten des Verbindungssystems angepasst werden, indem diese einfach in eine an die Größe der Ummantelung passende Spritzgussform gelegt und dort ummantelt werden. Ummantelungen die in einem Spritzgussverfahren hergestellt werden sind typischerweise nicht lösbar und dauerhaft.

Vorzugsweise weist die Ummantelung eine rohrförmige, rechteckige, polygonale, ovale oder zylindrische äußere Form auf. Solche Formen von Ummantelungen sind in einer Spritzgussform einfach vorzugeben und ermöglichen auch, dass die ummantelten Bauteile an der Ummantelung festgehalten werden, z.B. mittels einer weiteren Festhalteeinrichtung, da z.B. bei der Verwendung von Schläuchen in einem Chromatographiegerät die Schläuche im Gerät geführt werden müssen, aber nicht im Bereich der Schläuche gehalten werden sollen, da eine Klammerung der Schläuche zu einer unerwünschten Querschnittsveränderung führen würde.

In einer weiteren Ausführungsform weist der freie Endbereich des ersten Bauteils eine externe konusförmige Form und der freie Endbereich des zweiten Bauteils eine interne konusförmige Form auf. Solche konusförmigen Formen können einfach gefertigt werden und ermöglichen ein einfaches Zusammenfügen der Bauteile beim Aufbau der Verbindung des Verbindungssystems. Ferner gewährleisten solche konusförmigen Formen, dass die Bauteile gut aufeinander abgestimmt sind sowie eine weitestgehend verschleppungsfreie Verbindung ohne nennenswerte Querschnittsveränderung zwischen den Bauteilen.

In einer weiteren bevorzugten Ausführungsform weist das Verbindungssystem eine Hülse auf, die vorzugsweise eine Innenform hat, die komplementär zu einer Außenform des freien Endbereichs des zweiten Bauteils ist und diesen zumindest zum Teil umschließt. Somit kann der Flansch des zweiten Bauteils noch besser an den Flansch des ersten Bauteils gedrückt werden und eine bessere Verbindung zwischen den Bauteilen garantieren.

In einer bevorzugten Ausführungsform greift die Festhalteeinrichtung bzw. die zumindest eine Klammer in einer an der Hülse vorgesehenen Nut bzw. einer an der Hülse vorgesehenen Schulter an. Das Vorsehen einer solchen Nut bzw. Schulter ermöglicht nicht nur einen sicheren Zusammenhalt, sondern auch ein genaues Abstimmen der Länge der Bauteile aufeinander, so dass möglichst keine Verschleppungsbereiche beim Verbinden entstehen können.

In einer bevorzugten Ausführungsform verhindert die Hülse zumindest im Wesentlichen ein Verformen des freien Endbereichs des zweiten Bauteils beim Formen der Ummantelung. Dies gilt insbesondere dann, wenn die Ummantelung in einem Spritzgussverfahren hergestellt wird, da in einem solchen Spritzgussverfahren zweitweise recht hohe Drücke herrschen. Die Hülse übernimmt bei einem solchen Spritzgussverfahren auch eine Schutzfunktion, indem sie den darunterliegenden Flansch und zumindest einen Teil der Verbindung und/oder des Schlauchs an einem Verformen hindert und so gewährleistet, dass auch nach dem Spritzgussverfahren keine Querschnittsveränderungen bzw. Verschleppungsbereiche entstehen.

Nach einer weiteren Ausführungsform sind Dichtungsmittel zwischen dem ersten und dem zweiten Verbindungsbereich vorgesehen. Solche Dichtungsmittel sorgen für eine oder eine zusätzliche Abdichtung der Bauteile untereinander, bevor die Ummantelung vorgesehen wird bzw. für den Fall, dass keine Ummantelung verwendet wird. Solche Dichtungsmittel können z.B. einen Klebstoff, ein Silikondichtmittel oder einen O-Ring umfassen, der bzw. das zwischen den zwei Verbindungsbereichen eingefügt wird, um diese, vorzugsweise dichtend, miteinander zu verbinden.

Ein weiterer Aspekt der Erfindung betrifft ein Verfahren zum Verbinden von zwei fluidtransportierenden Bauteilen mit den Schritten:
- Zusammenstecken der Bauteile, vorzugsweise ein dichtendes Zusammenstecken der Bauteile;
- gegebenenfalls Fixieren der zusammengesteckten Bauteile; und
- Verbinden der zusammengesteckten Bauteile mittels einer im Spritzgussverfahren hergestellten Ummantelung. Solch ein Verfahren wird typischerweise so ausgeführt, dass die gegebenenfalls fixierten Bauteile in einer Spritzgussform platziert werden, die die Form der Ummantelung der Verbindung definiert, bevor die Bauteile mittels des Spritzgussverfahrens in der Spritzgussform ummantelt bzw. verbunden werden. Mittels eines solchen Verfahrens kann ein Verbindungssystem relativ einfach und kostengünstig hergestellt werden.

Die Erfindung wird nachfolgend anhand von Ausführungsbeispielen unter Bezugnahme auf die Zeichnungen näher erläutert, in denen zeigen:
- Fig. 1A: schematisch die einzelnen Komponenten eines erfindungsgemäßen Verbindungssystems vor dem Zusammenbau,
- Fig. 1B: die einzelnen Komponenten des erfindungsgemäßen Verbindungssystems gemäß Fig. 1A nach dem Zusammenbau,
- Fig. 1C: eine Detailzeichnung des Verbindungssystems der Fig. 1B,
- Fig. 2A: einen ersten Flansch eines erfindungsgemäßen Verbindungssystems,
- Fig. 2B: eine Detailzeichnung des Bereichs A des Flansches gemäß Fig. 2A,
- Fig. 3A: einen zweiten Flansch eines erfindungsgemäßen Verbindungssystems,
- Fig. 3B: eine Detailzeichnung des Bereichs A des Flansches gemäß Fig. 3A,
- Fig. 3C: eine weitere Detailzeichnung des Bereichs A des Flansches gemäß Fig. 3A,
- Fig. 4A: eine Seitenansicht einer Hülse eines erfindungsgemäßen Verbindungssystems ,
- Fig. 4B: einen Schnitt durch die Seitenansicht der Hülse gemäß Fig. 4A entlang der Schnittlinie A-A,
- Fig. 5: einen schematischen Aufbau eines weiteren erfindungsgemäßen Verbindungssystems,
- Fig. 6A: eine Seitenansicht eines erfindungsgemäßen Verbindungsystems,
- Fig. 6B: eine zum Teil geschnittene Darstellung des Verbindungssystems gemäß Fig. 6A entlang der Schnittlinie A-A der Fig. 6A, und
- Fig. 6C: eine Detailzeichnung des Bereichs B der Fig. 6B, die eine zum Teil geschnittene Darstellung des Verbindungssystems gemäß Fig. 6A zeigt.

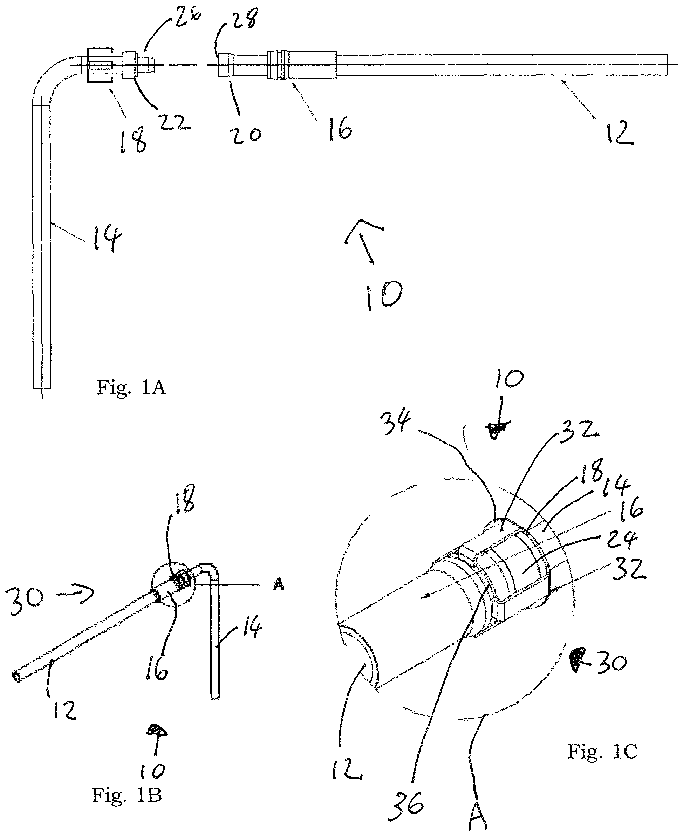

Bezug nehmend auf die Ausführung gemäß den Figuren 1A, 1B und 1C, zeigt die Fig. 1A schematisch ein Beispiel eines Verbindungssystems 10 vor dem Zusammenbau der einzelnen Komponenten 12, 14, 16, 18. Das Verbindungssystem 10 umfasst zwei fluidführende Bauteile 12, 14, insbesondere einen Schlauch 12, der einen Flansch 20 (hier eine sogenannte Schlauchaufweitung 20) aufweist, der mit einem Flansch 22 (hier ein sogenannter Halteflansch 22) eines Anschlussteils 14 (hier eine sogenannte Hohlnadel) beim Zusammenbau kooperiert bzw. zusammenarbeitet. Des Weiteren umfasst das Verbindungssystem der Fig. 1A eine Hülse 16 und eine Festhalteeinrichtung 18.

Beim Zusammenbau der Komponenten 12, 14, 16, 18 der Fig. 1A zu dem Verbindungssystem 10 der Fig. 1B wird der Flansch 20 bzw. die Schlauchaufweitung des Schlauches 12 über den Halteflansch 22 der Hohlnadel 14 geführt, bis der Flansch 20 des Schlauches 12 an einem Anschlag 24 zum Anliegen kommt. Somit wird ein freier Endbereich 26 des einen Bauteils 14 in einen freien Endbereich 28 des zweiten Bauteils 12 gesteckt.

In der vorliegenden Erfindung ist ein Flansch als ein vorgefertigter Anschlussbereich eines Bauteils zu verstehen, mittels dem ein weiteres Bauteil an dem vorgefertigten Anschlussbereich z.B. über einen weiteren Flansch angeschlossen werden kann. Das heißt, dass ein vorgefertigter Anschlussbereich eines Schlauches 12, z.B. in der Form einer Schlauchaufweitung ein Flansch 20 eines Bauteils 12 sein kann, während eine Hohlnadel 14, die in diese Schlauchaufweitung steckbar ist, ebenfalls einen Flansch 22 aufweisen kann. Mit anderen Worten sind die zwei Anschlussbereiche der Bauteile 12, 14 so ausgelegt, dass sie miteinander kooperieren, um eine Verbindung zwischen den zwei Bauteilen zu erzielen.

Der Flansch 20 des Schlauches 12 ist am oder hinter dem freien Endbereich 28 des zweiten Bauteils 12 ausgebildet und der Flansch 22 der Nadel 14 ist vom freien Endbereich 26 des ersten Bauteils 14 zurückgesetzt, so dass diese zusammengesteckt werden können. Zum Fixieren der Verbindung 30 gemäß Fig. 1B wird eine Hülse 16 über den äußeren Bereich des Flansches 20 des Schlauches 12 geführt und mittels einer Festhalteeinrichtung 18, die an bzw. hinter dem Flansch 22 der Nadel 14 angeordnet ist, festgehalten. Somit wird der Schlauch 12 in axialer Richtung an der Nadel 14 gehalten.

Wie der Detailzeichnung der Fig. 1C zu entnehmen ist, wird die Festhalteeinrichtung 18 durch eine Klammer gebildet, die mit Krallen 32 versehen ist, die sich von einem Ringteil 34 in Richtung des zweiten Flansches 20 erstrecken. Im Speziellen kommen hier vier Krallen 32 zum Einsatz, von denen jedoch zwei durch die Hülse 16 in der Zeichnung verdeckt sind. Das Ringteil 34 ist im vorliegenden Beispiel hinter dem Flansch 22 des Bauteils 14 axial verschiebbar angeordnet.

Das Ringteil 34 könnte jedoch auch integral am Flansch 22 hergestellt werden, so dass das Ringteil 34 zum Flansch 22 relativ gesehen nicht beweglich ist (nicht gezeigt). Alternativ könnte eine Klammer, gegebenenfalls mit mehreren Krallen (eine Festhalteeinrichtung) auch an der Hülse 16 angebracht sein, die hinter dem Flansch 22 der Nadel greifen (auch nicht gezeigt), um die Hülse 16 an dem Flansch zu fixieren, so dass auch hier der Schlauch 12 insbesondere fixierend und dichtend mit der Nadel 14 verbunden wird.

Diese Konstruktion dient dazu, das zweite Bauteil 12 - z.B. einen Schlauch oder ein Rohr - bzw. den Flansch 20 des Schlauches 12 am Flansch 22 der Hohlnadel 14 zu fixieren. Zu diesem Zweck ist die Hülse 16 vorgesehen, die axial von hinten über den Flansch 20 verschiebbar angeordnet und von den Krallen 32 der Festhalteeinrichtung 18 befestigt bzw. hintergriffen wird. Die Krallen 32 der Festhalteeinrichtung 18 greifen hierzu in einer an der Hülse 16 vorgesehenen Nut 36 an. Anstelle einer Nut 36 könnte z.B. auch eine Schulter an der Hülse 16 vorgesehenen sein (nicht gezeigt), hinter der die Krallen 32 einschnappen und im gespannten Zustand die Hülse 16 über dem zweiten Flansch 20 halten.

Sollte ein Rohr anstelle eines Schlauches 12 verwendet werden, könnte die Nut 36 bzw. Schulter auch direkt außen am Rohr oder direkt an einem am Rohr angebrachten Flansch (beides nicht gezeigt) vorgesehen sein, so dass keine Hülse 16 benötigt werden würde, um das zweite Bauteil 12 am ersten Bauteil 14 zu fixieren. Auch könnte die Festhalteeinrichtung 18 an der Hülse 16 bzw. am Rohr befestigt sein und hinter dem Anschlag des Flansches 22 des ersten Bauteils 14 angreifen.

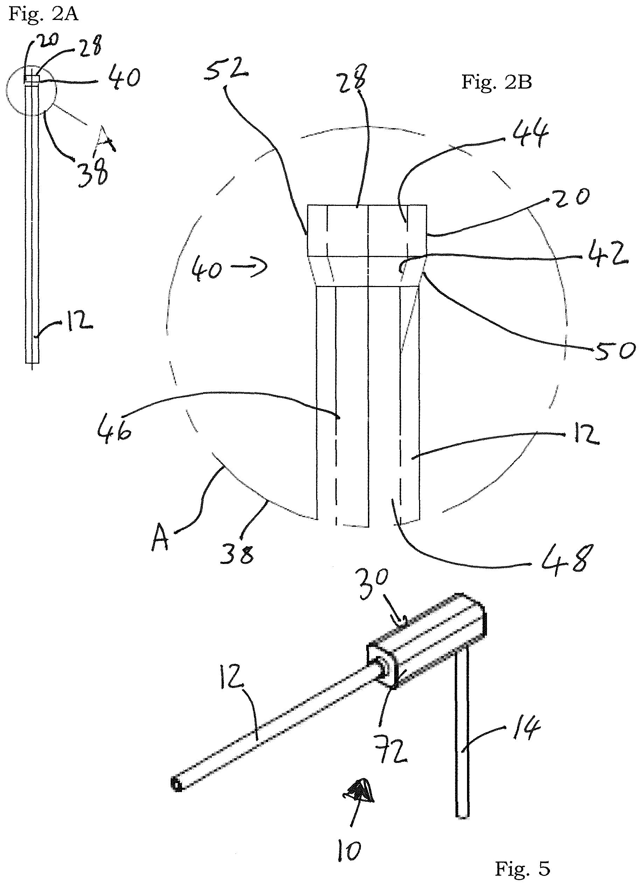

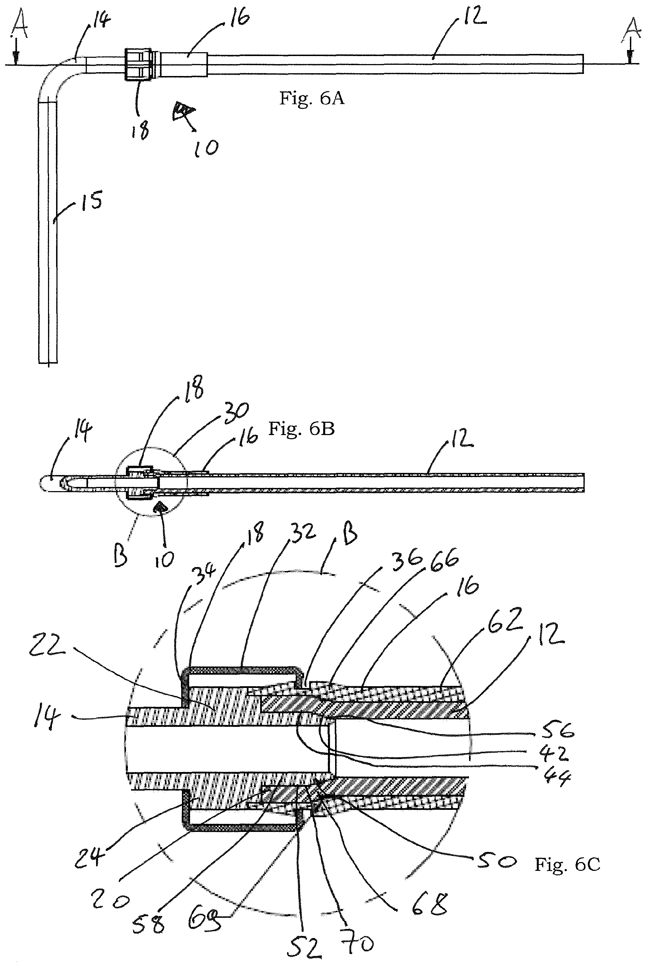

Eine mögliche Auslegung des Schlauchs 12 der Fig. 1A bis 1C ist schematisch in der Fig. 2A im Detail gezeigt. An einem Ende 38 des Schlauchs 12 ist ein Verbindungsbereich 40 vorgesehen, der einen Flansch 20 (Schlauchaufweitung) hat, der an der freien Stirnseite 28 des Bauteils 12 beginnt. Wie der Detailzeichnung der Fig. 2B zu entnehmen ist, hat der Flansch 20 eine interne Form, die einen konusförmigen Bereich 42 und einen rohrförmigen Bereich 44 größeren Durchmessers umfasst und komplementär zur äußeren Form des Flansches 22 des ersten Bauteils 14 ist (siehe z.B. Fig. 6C). Der konusförmige Bereich 42 ist zwischen dem zylindrischen Bereich 44 größeren Durchmessers und einem rohrförmigen Bereich 46 kleineren Durchmessers angeordnet. Der Durchmesser des Bereichs 46 kleineren Durchmessers entspricht im Wesentlichen dem Durchmesser eines Strömungskanals 48 des Schlauches 12 und (in Bezug auf die Fig. 6C) im Wesentlichem dem Durchmesser eines Strömungskanals (nicht gezeigt) der Hohlnadel 14.

Des Weiteren zeigen die Fig. 2A und 2B, dass das Schlauchende 38 benachbart der freien Stirnseite 28 des Verbindungsbereichs 40 auch eine äußere Form aufweist, die von einer typischen Schlauchform abweicht. Im Speziellen umfasst der Flansch 20 des Schlauches 12 auch einen externen konusförmigen Bereich 50 und einen externen rohrförmigen Bereich 52 größeren Durchmessers, wobei der Durchmesser des externen rohrförmigen Bereichs 52 größeren Durchmessers größer ist als der äußere Schlauchdurchmesser.

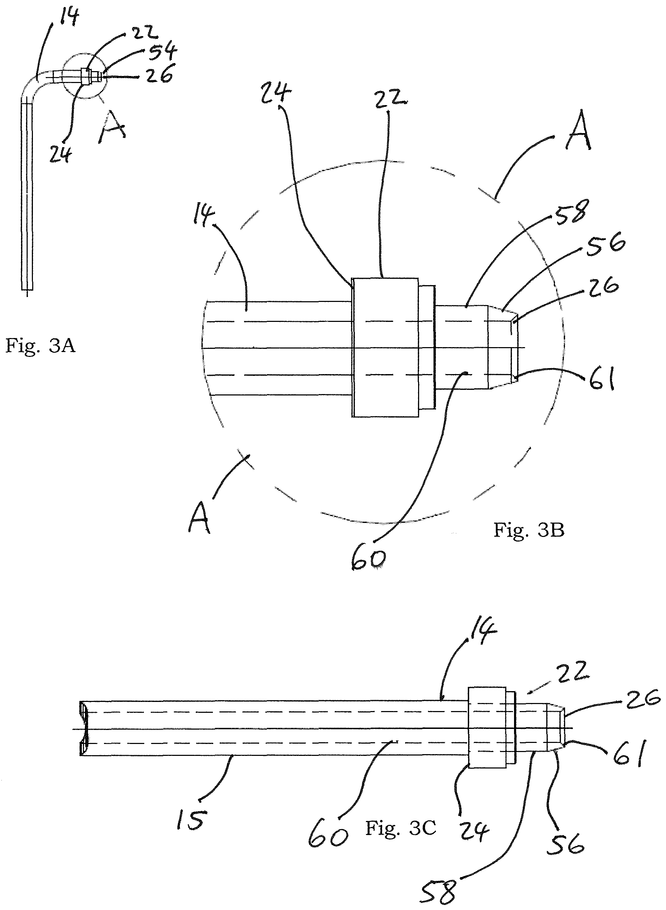

Eine mögliche Auslegung der Hohlnadel 14 der Fig. 1A bis 1C ist schematisch in der Fig. 3A im Detail gezeigt. Die Fig. 3A und 3B zeigen eine Hohlnadel 14 als erstes Bauteil. Auch hier ist an einem Ende 26 der Nadel 14 ein Verbindungsbereich 54 vorgesehen, der einen Flansch 22 hat, der vom freien Endbereich 26 des Bauteils 14 zurückgesetzt ist. Wie aus der Fig. 3B ersichtlich hat der Flansch 22 eine äußere Form, die einen externen konusförmigen Bereich 56 und einen externen rohrförmigen Bereich 58 umfasst. Der Konuswinkel des externen konusförmigen Bereichs 56 des Bauteils 14 der Fig. 3B entspricht der Steigung des internen konusförmigen Bereichs 42 des Bauteils 12 der Fig. 2B.

Der Durchmesser des Bereichs 58 der Nadel 14 ist vorzugsweise geringfügig größer oder zumindest im Wesentlichen gleich dem Durchmesser des Bereichs 44 größeren Durchmessers des Bauteils 12 gemäß Fig. 2B. Dadurch dass der Durchmesser des Bereichs 58 größeren Durchmessers vorzugsweise geringfügig größer oder zumindest im Wesentlichen gleich dem Durchmesser des Bereichs 44 größeren Durchmessers der Schlauchaufweitung des Schlauchs 12 ist, wird sichergestellt das der konusförmige Bereich 42 der Schlauchaufweitung an den konusförmigen Bereich 56 der Nadel gepresst wird, da der Bereich größeren Durchmessers 58 den Bereich größeren Durchmessers sozusagen aufspreizt und eine kraftschlüssige Verbindung herbeiführt, wobei die Verbindung abgedichtet wird. Mit anderen Worten hat der rohrförmige Bereich 44 des Flansches 20 am Ende des Schlauches 12 bevorzugt einen Innendurchmesser, der kleiner als der Außendurchmesser des rohrförmigen Bereich 58 des Flansches 22 der Hohlnadel 14 ist, um den Schlauch an die Nadel zu pressen, so dass keine Totwasserbereiche entstehen können bzw. um die Entstehung dieser zumindest zu minimieren.

Die in der Fig. 3A und B gezeigte Hohlnadel 14 hat ebenfalls einen Strömungskanal 60, dessen Durchmesser im Wesentlichem dem Durchmesser des Strömungskanals 48 des Schlauchs 12 der Fig. 2B entspricht. Der Durchmesser des Strömungskanals 60 der Nadel 14 kann aber auch vom Durchmesser des Strömungskanals 48 abweichen (nicht gezeigt), z.B. entweder größer oder kleiner sein als der Durchmesser des Strömungskanals 48 des Schlauches 12.

Bei Verwendung von zwei Strömungskanälen 48, 60, die einen im Wesentlichen gleichen Durchmesser aufweisen, wird beim Einstecken des ersten Bauteils 14 in das zweite Bauteil 12 somit eine Verbindung 30 zwischen den zwei Bauteilen 12, 14 geschaffen, die keine Querschnittsveränderung des Strömungskanals 48, 60 im Bereich der Verbindung 30 herbeiführt und auch keine nennenswerten Verschleppungsbereiche hat, da die Durchmesser der Strömungskanäle 48, 60 gleich sind und die zueinander komplementären Formen der jeweiligen konusförmigen Bereiche 42, 56 keine nennenswerten Verschleppungsbereiche bilden, wenn die zwei Bauteile aneinander angeschlossen werden.

Am Ende des Flansches 22 ist ein Übergangsbereich 61 zu sehen, der verhindert, dass der Flansch 20 beim Verbinden der Bauteile 12, 14 durch den Flansch 22 beschädigt wird. Ferner verhindert bzw. minimiert dieser Übergangsbereich 61 die Schaffung von ungewünschten Totwasserbereichen im Bereich der Verbindung zwischen den beiden Flanschen 20, 22. Der Übergangsbereich 61 bewirkt, dass das Material des Schlauchs 12 im Bereich des Übergangsbereichs 61 beim Verbinden der Bauteile 12, 14 so bearbeitet wird, dass es in einen Freiraum, der durch den Übergangsbereich 61 geschaffen wird, fliesen kann. Somit wird im Wesentlichen verhindert, dass Querschnittsveränderungen der Strömungskanäle 48, 60 im Bereich des Übergangsbereichs 61, sprich am Übergang zwischen den zwei Bauteilen 12, 14, geschaffen werden, die zu Verschleppungsbereichen und/oder Querschnittsveränderungen im Bereich des Übergangs der Strömungskanäle 48, 60 ineinander führen würden.

In dem speziellen Beispiel der Fig. 3B hat der Übergangsbereich 61 die Form einer Fase (auch als Kantenbruch bekannt). Es sind auch andere Formen von Übergangsbereichen 61 denkbar, sofern diese einen Übergang zwischen den zwei Bauteilen 12, 14 ermöglichen, der im Wesentlichen frei von Querschnittsveränderungen und/oder Totwasserbereichen ist.

Die Fig. 3C zeigt eine weitere Detailzeichnung des Bereichs A des Flansches gemäß Fig. 3A. Die Hohlnadel 14 der Fig. 3C hat einen Schaft 15 und weist ebenfalls den rohrförmigen Bereich 58 auf, der einen Durchmesser hat, der kleiner als der Außendurchmesser des Schaftes 15 der Hohlnadel 14 ist. Im Prinzip kann der Durchmesser des rohrförmigen Bereichs 58 gleich dem Außendurchmesser des Schaftes 15 der Hohlnadel 14 ausgewählt sein.

Der Durchmesser des rohrförmigen Bereichs 58 und des Außendurchmesser des Schaftes 15 und somit die Größe des Flansches 20 wird in der Praxis so ausgewählt, dass die Größe der einzelnen Komponenten wie der Schlauch 12, die Hohlnadel 14 und die Hülse 16 auf das jeweilige Einsatzgebiet abgestimmt sind. D.h. im Wesentlichen wird die Größe der Hohlnadel 16 so ausgewählt, dass eine zu fördernde Flüssigkeitsmenge bei dem vorgesehenen Druck gefördert werden kann.

Die Hohlnadel 14 mit dem Flansch 20 kann z.B. dadurch hergestellt werden, indem der Flansch 20 mit dem Anschlag 24 als separates Bauteil gefertigt wird und auf dem Schaft einer Hohlnadel platziert und dort mittels Kleben, Pressen, Schweißen, Löten oder einem ähnlichem Verfahren bzw. einer Kombination solcher Verfahren befestigt wird. Sollte wie in dem Beispiel der Fig. 3C der rohrförmige Bereich 58 einen kleineren Durchmesser aufweisen als der Außendurchmesser des Schaftes 15, kann dieser kleinere Durchmesser z.B. durch Drehen in einer Drehbank oder ähnlichem erzeugt werden. Alternativ kann die Hohlnadel schon mit zwei verschiedenen Außendurchmessern und dem konusförmigen Bereich 56 hergestellt werden, so dass vorgefertigte Flansche 20 einfach auf den Bereich des kleineren Außendurchmessers 58 der Hohlnadel 14 geschoben und dort mit der Hohlnadel 14 fluiddichtend verbunden werden können.

Die innere Form bzw. die äußere Form der komplementären Bauteile 12, 14 muss nicht die Form eines Konus aufweisen, kann z.B. auch eine stufige oder gerundete Form aufweisen, sofern die Form der Innenfläche des Flansches 20 des zweiten Bauteils 12 komplementär zu der Form der Außenfläche des Flansches 22 des ersten Bauteils 14 ist und eine stufenfreie Verbindung zwischen den Flanschen 20, 22 ermöglicht wird, so dass keine Verschleppungsbereiche beim Verbinden geformt werden und auch keine Querschnittsveränderung im Bereich der Verbindung 30 entsteht.

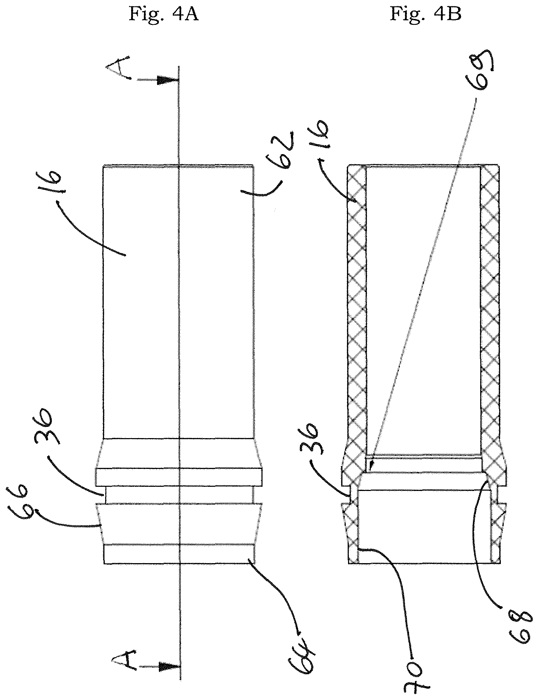

Um die Verbindung 30 zwischen dem ersten Flansch 22 und dem zweiten Flansch 20 zu fixieren, wird z.B. mittels einer Hülse 16 (siehe Fig. 1A und Fig. 4A) und zumindest einer Klammer 32 der Flansch 20 des zweiten Bauteils 12 am Flansch 22 des ersten Bauteils 14 fixiert (siehe Fig. 6C).

Fig. 4A zeigt eine Draufsicht auf die äußere Form einer solchen Hülse 16. An einem ihrer Enden 62 weist die Hülse 16 einen im Wesentlichen durchgehenden Durchmesser auf und an ihrem anderen Ende 64 einen Bereich 66 mit erweitertem Durchmesser, in dem eine umlaufende Nut 36 vorgesehen ist. Die Hülse 16 wird axial über den Flansch 20 des Bauteils 12 geschoben (siehe Fig. 1C) und nach dem Anschlagen am Anschlag 24 des ersten Bauteils 14 dort mittels einer Festhalteeinrichtung 18 fixiert. Die Festhalteeinrichtung 18 greift an der an der Hülse 16 vorgesehenen Nut 36 an. Um den Flansch 20 des Schlauches 12 fixieren zu können, weist die Hülse 16 an dem Ende 64, wo die Nut 36 vorgesehen ist, eine Innenform auf, die komplementär zur Außenform des Flansches 20 (z.B. der Schlauchaufweitung der Fig. 2B) des zweiten Bauteils 12 ist.

In dem Beispiel der Fig. 4A und 4B umfasst die Hülse einen internen konusförmigen Bereich 68 und einen internen rohrförmigen Bereich 70 größeren Durchmessers, wobei der Durchmesser des rohrförmigen Bereichs 70 größeren Durchmessers größer ist als der äußere Schlauchdurchmesser. Dabei entspricht der Konuswinkel des konusförmigen Bereichs 68 dem Konuswinkel des externen konusförmigen Bereichs 50 des zweiten Bauteils 12, und auch der Durchmesser des rohrförmigen Bereichs 70 größeren Durchmessers ist an den Durchmesser des rohrförmigen Bereichs 52 größeren Durchmessers des zweiten Bauteils 12 angepasst, so dass die Hülse 16 eine Fixierung des Schlauches 12 an der Nadel 14 gewährleisten kann.

Ferner umfasst die Hülse 16 eine Nase 69 die am Ende des konusförmigen Bereichs 68 angeordnet ist. Die Nase 69 ist dazu ausgebildet, sich mit dem Schlauch 12 zu verkeilen, um das Herausziehen des Schlauches 12 aus der Hülse 16 zu erschweren bzw. zu vermeiden. Die Nase 69 hat sozusagen die Funktion eines Widerhakens, der ein ungewolltes Herausziehen des Schlauches 12 aus der Verbindung 30 vermeidet.

Die innere Form der Hülse 16 bzw. die äußere Form des komplementären Bauteils 12 muss nicht die Form eines Konus aufweisen, kann z.B. auch eine stufige oder gerundete Form aufweisen, sofern die Form der Außenfläche des Flansches 20 des zweiten Bauteils 12 komplementär zu der Form der Innenfläche der Hülse 16 ist. Ferner ermöglicht die Hülse 16 ein Fixieren des zweiten Bauteils 12 am ersten Bauteil 14, so dass nach dem Zusammenstecken der Bauteile 12, 14 die Verbindung 30 spielfrei gehalten wird. Ein Spiel in der Verbindung 30 zwischen den Bauteilen 12, 14 könnte unter Umständen zu ungewollten Verschleppungsbereichen im Bereich der Verbindung 30 führen und möglicherweise verhindern, dass die Bauteile 12, 14 gegeneinander abgedichtet sind.

Die Fig. 5 zeigt eine weitere Ausführungsform des Verbindungssystems 10. In dieser wird die Verbindung 30 zwischen den zwei ineinander bzw. aneinander gesteckten Verbindungsbereiche 40, 54 mittels einer Ummantelung 72 umgeben. Dabei wird die Verbindung 30 zwischen den zwei Bauteilen 12, 14 von außen fixiert und gegebenenfalls zusätzlich abgedichtet. Die Ummantelung 72 greift somit fixierend und gegebenenfalls dichtend am ersten und zweiten Bauteil 12, 14 an.

Sollten die zwei Bauteile mittels einer Festhalteeinrichtung 18, z.B. bestehend u.a. aus Krallen 32 und einer Hülse 16, zusätzlich fixiert sein, so wird die Ummantelung 72 auch diese Komponenten 16, 32 zumindest bereichsweise einschließen und so zu einer noch besseren Verbindung 30 zwischen den Bauteilen 12, 14 führen.

In dem Beispiel der Fig. 5 hat die Ummantelung 72 eine im Wesentlichen rechteckige Form. Die Form kann jedoch beliebig ausgewählt werden, sofern sie die Festigkeit und/oder Dichtigkeit der Verbindung 30 nicht negativ beeinträchtigt. Die Ummantelung 72 kann z.B. auch eine polygonale, rohrförmige, ovale oder zylindrische äußere Form aufweisen.

Die untereinander zu verbindenden Bauteile 12, 14 werden zum Formen der Ummantelung 72 in eine Spritzgussform gelegt (nicht gezeigt). Hierbei definiert die Form der Spritzgussform die äußere Form der Ummantelung 72. Danach wird in einem Spritzgussverfahren (wie dem Fachmann bekannt) das Material der Ummantelung 72 unter erhöhtem Druck und gegebenenfalls unter erhöhter Temperatur in die Spritzgussform gespritzt, so dass die darin enthaltenen Bauteile 12, 14 durch das Material der Ummantelung 72 umgeben werden und das Material der Ummantelung 72 in der Form erstarrt. Die so hergestellte Ummantelung 72 ist nicht lösbar mit den Bauteilen 12, 14 verbunden.

Wenn eines der Bauteile z.B. ein Schlauch 12 ist, kommt normalerweise eine Hülse 16 zum Einsatz, zum einen, um den Schlauch 12 besser am ersten Bauteil 14, z.B. eine Hohlnadel gemäß Fig. 3A, zu halten und zum anderen verhindert die Hülse 16 zumindest im Wesentlichen ein Verformen des Schlauchs 12 im Bereich der Ummantelung 72 beim Formen dieser.

Während eines Spritzgussverfahrens herrschen beim Einspritzen des Materials der Ummantelung 72 relativ hohe Drücke und gegebenenfalls hohe Temperaturen. Diese können den Schlauch 12 beim Übergang zur Nadel 14, sprich im Bereich des Flansches 20, verformen und zu einer Querschnittsveränderung und unter Umständen zum Entstehen von Verschleppungsbereichen führen. Dies wird durch die Hülse 16 im Wesentlichen vermieden. Die Hülse 16 verhindert jedoch nicht, dass der Schlauch 12 an den Flansch 22 des ersten Bauteils 14 gepresst wird, um eine Dichtung in diesem Bereich zwischen dem Schlauch 12 und der Hohlnadel 14 zu bewerkstelligen bzw. zu erhöhen.

Um die Dichtung in diesem Bereich noch besser zu gestalten, könnten auch weitere Dichtungsmittel (nicht gezeigt), wie z.B. ein O-Ring oder Klebstoff, zwischen den beiden Flanschen 20, 22 vorgesehen sein, z.B. zwischen den konusförmigen Bereichen 42, 56.

Die Fig. 6A bis 6C zeigen eine weitere Ausführungsform des Verbindungsystems 10. Fig. 6A zeigt eine Seitenansicht des Verbindungssystems 10, die Fig. 6B zeigt eine teilweise Schnittzeichnung der Ansicht gemäß Fig. 6A entlang der Schnittlinie A-A der Fig. 6A. Hierbei wird ersichtlich wie die Hülse 16 den Verbindungsbereich 40 des zweiten Bauteils 12 umschließt und diesen an das erste Bauteil 14 presst.

Die Detailzeichnung des Bereichs B ist in der Fig. 6C gezeigt. Hier erkennt man, dass die Hülse 16 eine Innenform aufweist, die komplementär zur Außenform des Flansches 20 des Schlauchs 12 ist und diesen an dem ersten Bauteil 14, der Hohlnadel, befestigt. Des Weiteren sieht man, dass durch die komplementäre Form der Flansche 20, 22 keine nennenswerten Verschleppungsbereiche zu erkennen sind. Solche unerwünschten Verschleppungsbereiche könnten zu einer Verfälschung einer Analyse führen, da sich hier Ablagerungen bilden könnten.

Dadurch wird erreicht, dass die zwei Strömungskanäle 48, 60 im verbundenen Zustand des Verbindungssystems 10 miteinander ausgerichtet sind, und mit einem konstanten Querschnitt oder einem sich kontinuierlich verjüngenden Querschnitt mindestens des einen Verbindungsbereiches ineinander übergehen. Hierbei wird keine Querschnittverengung an dem Übergang zwischen den Strömungskanälen 48, 60 der beiden Verbindungsbereiche 40, 54 erzeugt.

Die Verschleppungsbereiche werden durch einen konstanten Querschnitt oder einen sich kontinuierlich verjüngenden Querschnitt der Verbindungsbereiche 40, 54 bzw. der Flansche 20, 22 auch vollständig oder zumindest weitestgehend minimiert, so dass keine Totwasserbereiche entstehen, die im Betrieb der Verbindung 30 bzw. des Verbindungsystems 10 zu ungewollten Störungen führen.

Das Verbindungssystem 10 kann in der Praxis eine Vielzahl von verschiedenen Ausbildungen erfahren. Es können z.B. folgende Verbindungen realisiert werden:
a) eine Verbindung zwischen einem Schlauch 12, beispielsweise aus Teflon und einer sogenannten Hohlnadel 14 bestehend aus Metall, Keramik, Zirkonoxid, Saphir oder Kunststoff,
b) eine Verbindung zwischen zwei Schläuchen, die direkt aneinander gefügt werden,
c) eine Verbindung zwischen zwei Schläuchen, die über ein rohrförmiges Verbindungsstück aneinander befestigt werden,
d) eine Verbindung zwischen zwei rohrförmigen Teilen, die aus Metall, Keramik, Zirkonoxid, Saphir bzw. Kunststoff bestehen können,
e) eine Verbindung zwischen einem Schlauch oder einem Rohr und einem zapfenartigen Anschluss eines Gerätes.

Die Schläuche, die in der vorliegenden Erfindung zum Einsatz gelangen können, umfassen z.B. Silikonschläuche, Teflonschläuche, Gummischläuche, Kautschukschläuche usw.

In allen vorgenannten Beispielen kann mindestens der erste Flansch 22 integral mit einem der jeweiligen zu verbindenden Bauteile 12, 14 oder an einer Hülse 16 ausgebildet werden, die auf das Bauteil 12, 14 aufgeschoben oder aufgeschraubt wird. Ferner kann die Festhalteeinrichtung 18 durch eine Klammer 32 mit mehreren Krallen oder durch einzelne längliche Krallen 32 oder durch eine C-förmige Rohrklammer 32 gebildet werden.

### Bezugszeichenliste:

- 10: Verbindungssystem
- 12: Bauteil
- 14: Bauteil
- 15: Schaft
- 16: Hülse
- 18: Festhalteeinrichtung
- 20: Flansch
- 22: Flansch
- 24: Anschlag
- 26: Endbereich
- 28: freie Stirnseite
- 30: Verbindung
- 32: Kralle
- 34: Ringteil
- 36: Nut
- 38: Ende
- 40: Verbindungsbereich
- 42: konusförmiger Bereich
- 44: Bereich
- 46: Bereich
- 48: Strömungskanal
- 50: konusförmiger Bereich
- 52: Bereich
- 54: Verbindungsbereich
- 56: konusförmiger Bereich
- 58: Bereich
- 60: Strömungskanal
- 61: Fase
- 62: Ende
- 64: Ende
- 66: Bereich
- 68: konusförmiger Bereich
- 69: Nase
- 70: Bereich
- 72: Ummantelung

## Patentansprüche

1. Verbindungssystem (10) zum Verbinden von fluidführenden Bauteilen (12, 14), insbesondere ein Schlauchverbindungssystem, das ein erstes Bauteil (12) mit einem ersten Verbindungsbereich (54), wobei der erste Verbindungsbereich (54) einen ersten Flansch (22) umfasst, der von einem freien Endbereich (26) des ersten Bauteils (14) zurückgesetzt ist, ein zweites Bauteil (14) mit einem zweiten Verbindungsbereich (40) der mit dem ersten Verbindungsbereich (54) zur Ausbildung einer Verbindung (30) kooperiert, umfasst, wobei der zweite Verbindungsbereich (40) einen zweiten Flansch (20) umfasst, der am oder hinter einem freien Endbereich (28) des zweiten Bauteils (12) ausgebildet ist, wobei im ersten Verbindungsbereich ein erster Strömungskanal und im zweiten Verbindungsbereich ein zweiter Strömungskanal ausgebildet ist, wobei im verbundenen Zustand die zwei Strömungskanäle miteinander ausgerichtet sind und mit einem konstanten Querschnitt oder einem sich kontinuierlich verjüngenden Querschnitt mindestens des einen Verbindungsbereiches ineinander übergehen, d.h. zumindest im Wesentlichen ohne eine Diskontinuität ineinander übergehen, und wobei im Bereich der Verbindung (30) zumindest ein Teil des ersten und zumindest ein Teil des zweiten Verbindungsbereichs (54, 40) durch eine Ummantelung (72) fixierend und gegebenenfalls dichtend verbunden ist, **dadurch gekennzeichnet, dass** eine Festhalteeinrichtung (18) vorgesehen ist, die an oder hinter dem ersten Flansch (22) und/oder an oder hinter dem zweiten Flansch (20) angreift, die vorzugsweise durch zumindest eine Klammer (32) ausgebildet ist, um das erste und zweite Bauteil (12, 14) axial aneinander zu halten.

2. Verbindungssystem (10) nach Anspruch 1,
**dadurch gekennzeichnet, dass**
der erste Verbindungsbereich (54) in den zweiten Verbindungsbereich (40) einsteckbar ist.

3. Verbindungssystem (10) nach Anspruch 1 oder Anspruch 2,
**dadurch gekennzeichnet, dass**
**dadurch gekennzeichnet, dass**
eine Hülse (16) am zweiten Bauteil (12) hinter bzw. über dem zweiten Flansch (20) angeordnet ist und die die Festhalteeinrichtung bzw. die zumindest eine Klammer (32) an der Hülse (16) angreift.

4. Verbindungssystem (10) nach zumindest einem der vorstehenden Ansprüche,
**dadurch gekennzeichnet, dass**
die die zumindest eine Klammer an einem Ringteil (34) ausgebildet ist, das hinter dem ersten Flansch (22), vorzugsweise axial verschiebbar angeordnet ist.

5. Verbindungssystem (10) nach nach zumindest einem der vorstehenden Ansprüche,
**dadurch gekennzeichnet, dass**
die Ummantelung (72) ferner die Festhalteeinrichtung (18), die vorzugsweise durch zumindest eine Klammer (32) gebildet wird, zumindest bereichsweise ummantelt.

6. Verbindungssystem (10) nach zumindest einem der vorstehenden Ansprüche,
**dadurch gekennzeichnet, dass**
die Ummantelung (72) dichtend am ersten und zweiten Bauteil (12, 14) angreift und/oder die Festhalteeinrichtung (18), bzw. die zumindest eine Klammer (32) festhält.

7. Verbindungssystem (10) nach zumindest einem der vorstehenden Ansprüche,
**dadurch gekennzeichnet, dass**
die Ummantelung (72) im Spritzgussverfahren ausgebildet ist, und/oder dass
die Ummantelung (72) eine rohrförmige, rechteckige, polygonale, ovale oder zylindrische äußere Querschnittsform aufweist.

8. Verbindungssystem (10) nach zumindest einem der vorstehenden Ansprüche,
**dadurch gekennzeichnet, dass**
die Ummantelung (72) eine nicht lösbare Verbindung der ersten und zweiten Flansche bildet.

9. Verbindungssystem (10) nach zumindest einem der vorstehenden Ansprüche,
**dadurch gekennzeichnet, dass**
der freie Endbereich (26) des ersten Bauteils (14) eine externe konusförmige Form (56) aufweist und der freie Endbereich (28) des zweiten Bauteils (12) eine interne konusförmige Form (42) aufweist.

10. Verbindungssystem (10) nach zumindest einem der vorstehenden Ansprüche,
**dadurch gekennzeichnet, dass**
die Hülse (16) eine Innenform aufweist, die komplementär zu einer Außenform des freien Endbereichs (28) des zweiten Bauteils (12) ist und diesen zumindest zum Teil umschließt.

11. Verbindungssystem (10) nach zumindest einem der vorstehenden Ansprüche,
**dadurch gekennzeichnet, dass**
die Festhalteeinrichtung (18) bzw. die zumindest eine Klammer (32) in einer an der Hülse (16) vorgesehenen Nut (36) bzw. einer an der Hülse (16) vorgesehenen Schulter angreift, und/oder dass
die Hülse (16) ein Verformen des freien Endbereichs (28) des zweiten Bauteils (12) beim Formen der Ummantelung (72) zumindest im Wesentlichen verhindert, und/oder dass
Dichtungsmittel zwischen dem ersten und dem zweiten Verbindungsbereich (40, 54) vorgesehen sind.

12. Verbindungssystem (10) nach zumindest einem der vorstehenden Ansprüche,
**dadurch gekennzeichnet, dass**
die Festhalteeinrichtung (18) durch eine mit Krallen (32) versehene Klammer gebildet ist, wobei sich die Krallen (32) von einem Ringteil (34) in Richtung des zweiten Flansches (20) erstrecken.

13. Verbindungssystem (10) nach zumindest einem der vorstehenden Ansprüche,
**dadurch gekennzeichnet, dass**
an einem Ende des ersten Bauteils (14) ein Übergangsbereich (61), vorzugsweise in der Form einer Fase, vorgesehen ist, und/oder dass der Übergangsbereich (61) eine lokale, insbesondere eine konusförmige Aufweitung, vorzugsweise eine leicht konusförmige Aufweitung des Materials des zweiten Bauteils im Bereich eines Übergangs zwischen den zwei Strömungskanälen (48, 60) erlaubt.

14. Verfahren zum Verbinden von zwei Fluidtransportierenden Bauteilen (12, 14) gemäß einem der Ansprüche 1-13, mit den Schritten:
- Zusammenstecken der Bauteile (12, 14);
- Fixieren der zusammengesteckten Bauteile (12, 14) mittels einer Festhalteeinrichtung (18), die eine am zweiten Bauteil (12) hinter bzw. über dem zweiten Flansche (20) angeordnete Hülse (16) angreift, um das erste und zweite Bauteil (12, 14) axial aneinander zu halten; und
- Verbinden der zusammengesteckten Bauteile (12, 14) mittels einer im Spritzgussverfahrens hergestellten Ummantelung (72), wobei gegebenenfalls eine nicht lösbare Verbindung hergestellt wird.

15. Verfahren nach Anspruch 14, mit dem weiteren Schritt:
- Platzieren der gegebenenfalls fixierten Bauteile (12, 14) in einer Spritzgussform, die die Form der Ummantelung (72) der Verbindung (30) definiert, bevor die Bauteile (12, 14) mittels des Spritzgussverfahrens verbunden werden.

## Claims

1. A connecting system (10) for connecting fluid conducting components (12, 14), in particular a hose connecting system, that comprises a first component (12) having a first connection region (54),
wherein the first connection region (54) comprises a first flange (22) set back from a free end region (26) of the first component (14); a second component (14) having a second connection region (40) that cooperates with the first connection region (54) for the formation of a connection (30), wherein the second connection region (40) comprises a second flange (20) that is formed at or behind a free end region (28) of the second component (12), wherein a first flow passage is formed in the first connection region and a second flow passage is formed in the second connection region, wherein the two flow passages are aligned with respect to one another in the connected state and transition into one another with a constant cross-section or a continuously tapering cross-section of at least one of the connection regions, this means they transition into one another at least substantially without any discontinuity, and
wherein at least one part of the first and at least one part of the second connection region (54, 40) are connected to one another in the region of the connection (30) by means of a jacket (72) in a fixing manner and possibly in a sealing manner,
**characterized in that**
a securing device (18) is provided that engages at or behind the first flange (22) and/or at or behind the second flange (20) and that is preferably formed by at least one clamp (32) to axially secure the first and the second component (12, 14) with respect to one another.

2. A connecting system (10) in accordance with claim 1,
**characterized in that**
the first connection region (54) can be plugged into the second connection region (40).

3. A connecting system (10) in accordance with claim 1 or claim 2,
**characterized in that**
a sleeve (16) is arranged at the second component (12) behind or over respectively the second flange (20) and the securing device (18) and/or the at least one clamp (32) engages/engage at the sleeve (16).

4. A connecting system (10) in accordance with at least one of the preceding claims,
**characterized in that**
the at least one clamp is formed at a ring part (34) that is arranged behind the first flange (22), preferably in an axially displaceable manner.

5. A connecting system (10) in accordance with at least one of the preceding claims,
**characterized in that**
the jacket (72) further at least regionally covers the securing device (18) that is preferably formed by at least one clamp (32).

6. A connecting system (10) in accordance with at least one of the preceding claims,
**characterized in that**
the jacket (72) engages in a sealing manner at the first and the second component (12, 14) and/or secures the securing device (18) and/or the at least one clamp (32).

7. A connecting system (10) in accordance with at least one of the preceding claims,
**characterized in that**
the jacket (72) is formed in an injection-molded process;
and/or **in that**
the jacket (72) has a tubular, rectangular, polygonal, oval or cylindrical outer cross-sectional shape.

8. A connecting system (10) in accordance with at least one of the preceding claims,
**characterized in that**
the jacket (72) forms a non-releasable connection between the first and second flanges.

9. A connecting system (10) in accordance with at least one of the preceding claims,
**characterized in that**
the free end region (26) of the first component (14) has an external conical shape (56) and the free end region (28) of the second component (12) has an internal conical shape (42).

10. A connecting system (10) in accordance with at least one of the preceding claims,
**characterized in that**
the sleeve (16) has an internal shape that is complementary to an outer shape of the free end region (28) of the second component (12) and surrounds it at least in part.

11. A connecting system (10) in accordance with at least one of the preceding claims,
**characterized in that**
the securing device (18) and/or the at least one clamp (32) engages/engage in a groove (36) provided at the sleeve (16) and/or at a shoulder provided at the sleeve (16); and/or **in that**
the sleeve (16) at least substantially prevents a deformation of the free end region (28) of the second component (12) on forming the jacket (72); and/or in that
sealing means are provided between the first and the second connection region (40, 54).

12. A connecting system (10) in accordance with at least one of the preceding claims,
**characterized in that**
the securing device (18) is formed by a clamp that is provided with claws (32), with the claws (32) extending from a ring part (34) in the direction of the second flange (20).

13. A connecting system (10) in accordance with at least one of the preceding claims,
**characterized in that**
a transition region (61), preferably in the shape of a chamfer, is provided at an end of the first component (14); and/or **in that** the transition region (61) permits a local widening, in particular a conical widening, preferably a slightly conical widening of the material of the second component in the region of a transition between the two flow passages (48, 60).

14. A method of connecting two fluid transporting components (12, 14) in accordance with any one of the claims 1 to 13, comprising the steps of:
- plugging together the components (12, 14);
- fixing the plugged together components (12, 14) by means of a securing device (18) that engages at a sleeve (16) arranged at the second component (12) behind or over respectively the second flange (20) in order to axially secure the first and second components (12, 14) with respect to one another; and
- connecting the plugged together components (12, 14) by means of a jacket (72) produced in an injection-molded process, wherein a non-releasable connection may be produced.

15. A method in accordance with claim 14, comprising the further step of:
- placing the optionally fixed components (12, 14) into an injection mold that defines the shape of the jacket (72) of the connection (30) before the components (12, 14) are connected to one another by means of the injection-molded process.

## Revendications

1. Système de connexion (10) pour connecter des composants (12, 14) porteurs de fluide, en particulier un système de connexion de tuyau, qui comprend un premier composant (12) avec une première zone de connexion (54), dans lequel la première zone de connexion (54) comprend une première bride (22) qui est en retrait par une zone d'extrémité libre (26) du premier composant (14), un second composant (14) avec une seconde zone de connexion (40) qui coopère avec la première zone de connexion (54) pour former une liaison (30), la seconde zone de connexion (40) comprend une seconde bride (20) qui est forme sur ou derrière d'une zone d'extrémité libre (28) de la second composant (12), dans lequel sont formés un premier canal d'écoulement dans la première zone de connexion et un second canal d'écoulement dans la seconde zone de connexion,
dans lequel à l'état raccordé, les deux canaux d'écoulement sont alignés l'un par rapport à l'autre et se fondent l'un dans l'autre avec une section transversale constante ou décroissante en continu d'au moins une zone de connexion, c'est-à-dire se fondent au moins sensiblement sans discontinuité, et
dans lequel au moins une partie de la première et au moins une partie de la deuxième zone de raccordement (54, 40) sont reliées, dans la zone de connexion (30), de manière fixe et éventuellement étanche, par une enveloppe (72), **caractérisé en ce que**
il est prévu un dispositif de retenue (18) qui s'engage sur ou derrière la première bride (22) et/ou sur ou derrière la deuxième bride (20), qui est de préférence formé par au moins un clip (32) pour maintenir axialement les premier et deuxième composants (12, 14) l'un contre l'autre.

2. Système de connexion (10) selon la revendication 1,
**caractérisé en ce que**
la première zone de connexion (54) peut être insérée dans la deuxième zone de connexion (40).

3. Système de connexion (10) selon la revendication 1 ou 2,
**caractérisé en ce que**
**caractérisé en ce que**
un manchon (16) est disposé sur le deuxième composant (12) derrière ou au-dessus de la deuxième bride (20) et qui s'engage avec le dispositif de retenue ou l'au moins un clip (32) sur le manchon (16).

4. Système de connexion (10) selon au moins une des revendications ci-dessus,
**caractérisé en ce que**
l'au moins un clip est formé sur une partie annulaire (34) qui est disposée derrière la première bride (22), de préférence mobile axialement.

5. Système de connexion (10) selon au moins une des revendications ci-dessus,
**caractérisé en ce que**
l'enveloppe (72) enveloppe en outre le dispositif de retenue (18), qui est de préférence formé par au moins un clip (32), au moins dans des régions.

6. Système de connexion (10) selon au moins une des revendications ci-dessus,
**caractérisé en ce que**
l'enveloppe (72) s'engage de manière étanche sur le premier et le second composant (12, 14) et/ou retient le dispositif de retenue (18) ou l'un au moins clip (32).

7. Système de connexion (10) selon au moins une des revendications ci-dessus,
**caractérisé en ce que**
l'enveloppe (72) est formée par moulage par injection, et/ou **en ce que** l'enveloppe (72) a une section transversale extérieure tubulaire, rectangulaire, polygonale, ovale ou cylindrique.

8. Système de connexion (10) selon au moins une des revendications ci-dessus,
**caractérisé en ce que**
l'enveloppe (72) forme une connexion non détachable de la première et deuxième bride.

9. Système de connexion (10) selon au moins une des revendications ci-dessus,
**caractérisé en ce que**
la zone d'extrémité libre (26) du premier composant (14) a une forme externe conique (56) et la zone d'extrémité libre (28) du second composant (12) a une forme interne conique (42).

10. Système de connexion (10) selon au moins une des revendications ci-dessus,
**caractérisé en ce que**
le manchon (16) a une forme intérieure complémentaire d'une forme extérieure de la zone d'extrémité libre (28) du second composant (12) et l'enveloppe au moins partiellement.

11. Système de connexion (10) selon au moins une des revendications ci-dessus,
**caractérisé en ce que**
le dispositif de retenue (18) ou l'au moins un clip (32) s'engage dans une rainure (36) prévue sur le manchon (16) ou dans un épaulement prévu sur le manchon (16), et/ou **en ce que**
le manchon (16) empêche au moins sensiblement la déformation de la zone d'extrémité libre (28) du deuxième composant (12) pendant le formage de l'enveloppe (72), et/ou **en ce que**
des moyens d'étanchéité sont prévus entre lesdites première et deuxième zone de connexion (40, 54).

12. Système de connexion (10) selon au moins une des revendications ci-dessus,
**caractérisé en ce que**
le dispositif de retenue (18) est formé par un clip muni de griffes (32), les griffes (32) s'étendant depuis une partie annulaire (34) vers la seconde bride (20).

13. Système de connexion (10) selon au moins une des revendications ci-dessus,
**caractérisé en ce que**
une zone de transition (61), de préférence sous la forme d'un chanfrein, est prévue à une extrémité du premier composant (14), et/ou **en ce que** la zone de transition (61) permet une dilatation locale, en particulier une dilatation conique, de préférence une dilatation légèrement conique, du matériau du second composant dans la zone de transition entre les deux canaux d'écoulement (48, 60).

14. Procédé de connexion de deux composants (12, 14) de transport de fluide, selon une quelconque des revendications 1 - 13, comprenant les étapes suivantes :
- assembler les composants (12, 14) ;
- fixer des composants assemblés (12, 14) au moyen d'un dispositif de retenue (18) qui s'engage avec un manchon (16) disposé sur le deuxième composant (12) derrière et au-dessus de la deuxième bride (20), respectivement, afin de maintenir axialement les premier et deuxième composants (12, 14) l'un contre l'autre ; et
- connecter des composants assemblés (12, 14) au moyen d'une enveloppe (72) réalisé par moulage par injection, une connexion non démontable étant éventuellement réalisé.

15. Procédé selon la revendication 14, avec l'étape suivante :
- la mise en place des composants (12, 14) éventuellement fixés dans un moule d'injection définissant la forme de l'enveloppe (72) du joint (30) avant que les composants (12, 14) ne soient connectés par le procédé de moulage par injection.
